# EUROPEAN PATENT APPLICATION

(11) **EP 0 681 809 A1**
(43) Date of publication of application: **15.11.1995**
(21) Application number: 94117812.1
(22) Date of filing: 11.11.1994
(51) Int. Cl.: A61B 1/05

(54) **Stereo imaging assembly for endoscopic probe**

(30) Priority: 09.05.1994 US 239738
(71) Applicant: Welch Allyn, Inc., Skaneateles Falls, New York 13153-0050 (US)
(72) Inventor: Wood, Robert J., Syracuse, NY 13215 (US); Monroe, Richard A., Liverpool, NY 13088 (US); Walts, Connie R., Auburn, NY 13021 (US)
(74) Representative: Leineweber, Jürgen, Dipl.-Phys.

(57) **Abstract**

A miniature stereo imaging assembly for a laparoscope or similar probe (10) has an objective lens assembly (36) and an imager device (50). The objective lens (38,40) assembly has left and right lens cells contained in a housing (34) or block, with the lens cells having their optic axes (74,76) parallel. The imager device has two active imaging areas (56,58) formed side by side on a common substrate (54). The center-to-center spacing (84) between the active imaging areas is somewhat larger than the pupil distance (78) between the lens cell optic axes.

## Description

### Background of the Invention

This invention relates to probes of the type in which a miniature video camera is mounted at a distal viewing head of an elongated insertion tube. The invention is more particularly concerned with an arrangement of the miniature video camera which produces left-eye and right-eye stereo images using a unitary video imaging device and a consolidated objective lens assembly.

Recently, interest has increased in the use of video instruments for surgical applications, such as video laparoscopes, to permit a surgeon to carry out a procedure with minimal intervention in the patient. An example of one such video instrument is a laparoscope for performing surgery in the abdominal cavity, where the instrument is inserted through a small incision. Unfortunately, a video laparoscope or other optical laparoscope provides only a two-dimensional view of the area where surgery is to be performed. Consequently, there is an interest in stereo laparoscopy to aid the surgeon in identifying and repairing or removing tissues in question. For similar reasons, interest has recently increased in remote imaging of industrial process, such an inspection of heat exchanger tubes or of turbine engines, where stereoscopic imaging could be employed to advantage.

With a conventional monoscopic viewing system, depth and distance are difficult to gauge. It is usually necessary for the surgeon to bring the distal tip of the laparoscope into contact with a tissue or organ to gain precise knowledge of its location. Stereo imaging systems would eliminate this by providing visual gauging axial distance. Surgery becomes faster, because time to determine location is reduced approximately in half. Surgery also becomes safer because the surgeon does not have to touch the surgical instrument's tip to an object to establish a reference point. Moreover, stereoscopic viewing capability makes it easier for the surgeon to pick out features of organs.

However, in all previous stereoscopic systems, two monoscopic images are generated from two separate optical or video camera systems. The images are then displayed separately to the left and right eyes of the surgeon or other observer, giving the perception of three dimensional imaging. The two separate images must be created with the same magnification, orientation, focus, and optical qualities. This presents a significant manufacturing problem due to tolerance, repeatability and assembly. The cost of such an instrument would far exceed two times the cost of a standard, two dimensional video imager. Two sets of optical lens assemblies, two cameras, and two electronic imagers that are employed, must be exactly matched and aligned to the maximum extent possible in order to display an acceptable stereoscopic pair of images on a video monitor. Moreover, because two separate camera systems are required, a three-dimensional imaging laparoscope or other endoscope or borescope would be significantly bulkier and heavier than a corresponding two-dimensional imaging instrument.

At the time being, full-color video borescopes and endoscopes are well known, and have been described, for example, in Danna et al. U.S. Pat. No. 4,491,365, Danna et al. U.S. Pat. No. 4,539,586, and Longacre et al. U.S. Pat. No. 4,523,224. The latter describes a color-sequential system in which primary color light is supplied sequentially over a fiber optic bundle to illuminate a target area.

A stereo imaging system that employs a single lens group and a single imager in a borescope or endoscope is described in U.S. Pat. No. 5,222,477. There, a wide-angle focusing lens assembly, with an effective aperture of f/2 or wider is teamed with an aperture plate that has left and right pupils on opposite sides of the optic axis of the lens assembly. The two pupils are shuttered alternately, and a left image and right image are alternately focused on the imager. The output signal of this device is processed and left and right views are presented on a monitor or other viewing device, so the object can be viewed stereoscopically.

In this system, miniature liquid crystal shutters, or other miniature shutters that can be operated at high speed, are required for each pupil. Also, because the images are formed through an off-axis region of the lens group, some distortion can result.

### Object and Summary of the Invention

Accordingly, it is an object of this invention to provide a simple and efficient stereo imaging assembly for a borescope or endoscope, e.g. for a rigid laparoscope, and which overcomes the drawbacks of the prior art.

It is another object of this invention to provide a stereoscopic imaging and viewing system for a laparoscope or other inspection instrument, for which matching and alignment problems are eliminated.

It is a further object to provide a stereo imaging assembly which is compact and rugged, and which can be economically constructed.

It is a still a further object of the invention to provide a stereo imaging assembly in which the left and right display images can be electronically aligned.

According to one aspect of this invention, the imaging assembly, designed to be situated at the distal tip of an insertion tube, has an objective lens assembly and an electronic dual imaging device. The objective lens assembly has left and right lens cells contained in a common lens housing, with the lens cells disposed side by side having respective optic axes parallel to one another and to the insertion tube axis at the distal tip. The lens cells have their optic axes separated from one another by a predetermined pupil distance. The imager device has left and right active images formed side by side on a common substrate in a common image plane. The image areas have image centers that are separated from each other by a predetermined center-to-center spacing that is at least slightly greater than the corresponding pupil distance. This establishes a converging point for the left and right images that is a short distance in advance of the distal tip. The images can be processed and electronically moved laterally out or in, so that the images converge at different distances as necessary. This feature can be used to compute distance automatically.

In one specific embodiment, where the insertion tube is 12 mm in diameter, the lens cells have a diameter of 0.175 inches and a focal distance of 1.5 inches. The lens housing has dimensions of about 0.452 inches by 0.304 inches. The imager active areas have a center-to-center spacing of about 0.210 inches, and the pupil distance between the two lens cells is about 0.190 inches.

Placing the active images together on one substrate and in a common plane ensures matched performance for left and right images. The flat imager plane, i.e. disposed across the tube axis rather than being angled in, avoids optical distortion, such as "fisheye" elongation at the image edges.

Having dual image areas on the single imaging device permits size constraints to be observed, and ensures alignment of the image areas in three dimensions. Disposing both left and right lens cells in parallel, side-by-side configuration in a common block or housing ensures precise alignment of the focusing optics.

Both left and right images can be reproduced, as alternating fields, on a monitor or video screen. The two images can be observed stereoscopically using infrared controlled viewing glasses.

As mentioned before, the offset between the left and right images can be adjusted electronically, for example, as target distance changes, to produce a correct convergence point or image crossing point. The amount of image offset needed to produce convergence can be used for computation of target distance.

Fiber optic bundles carry light through the insertion tube to its distal end. The fiber optic bundle fans out and is fitted into crescent-shaped passages above and below the lens housing.

The use of the stereo imaging system of this invention cuts surgery time by one half, and is both safer for the patient and more versatile for the surgeon.

The above and many other objects, features, and advantages of this invention will become more fully appreciated from the ensuing description of the preferred embodiment which should be read in connection with the accompanying Drawing.

### Brief Description of the Drawing

Fig. 1 is a perspective view of an endoscope or borescope according to one embodiment of this invention.

Figs. 2 and 3 are partial perspective views of the imaging assembly of one preferred embodiment of the invention, Fig. 2 being partly cut away.

Fig. 4 is a sectional elevation of the stereo imaging assembly of this embodiment.

Figs. 5 and 6 are a top plane and side elevation of the imager device of this embodiment.

### Detailed Description of the Preferred Embodiment

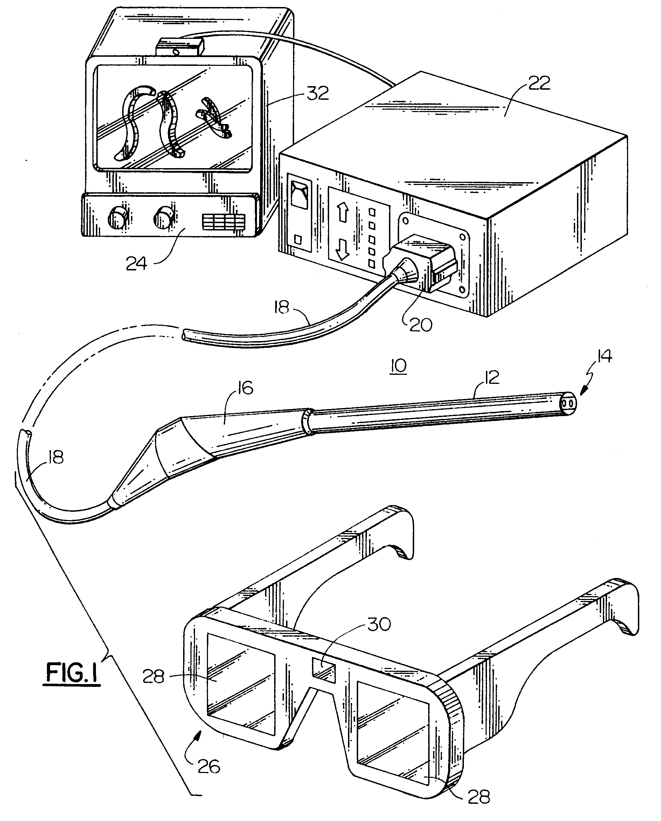

With reference to the Drawing, Fig. 1 shows a stereo video laparoscope assembly 10, in this case having an elongated rigid insertion tube 12 with a stereoscopic video camera 14 situated at its distal tip. The proximal end of the tube is affixed into a handle 16, and a flexible umbilical 18 continues proximally from the handle to a modular connector 20. The umbilical 18 contains electrical conductors that carry video output signal from the camera 14 to video processing circuitry contained in the modular connector 20, and also contains an optical fiber bundle that carries illumination to the distal tip of the insertion tube 12 for illuminating a target located distally of the camera 14.

The connector 20 plugs into a socket on a power supply and light source unit 22, which contains a lamp that supplies light to the proximal end of the fiber bundle. The unit 22 contains a wiring harness that carries the processed video signals to a video outlet that can be coupled to a video monitor 24. In this case, the monitor is adapted to operate at a field rate of 120 fields per second, presenting the left and right images alternately.

For this particular embodiment an electronically shuttered mask or spectacle set 26 is employed. The mask 26 has left and right eyepieces 28, 28, which can be gated or shuttered electrically. The mask has internal electronics for shuttering these eyepieces, and carries an infrared sensor 30 for toggling the eyepieces to permit viewing of left and right images alternately. An infrared transmitter 32 located on or adjacent the monitor 24 is synchronized, e.g., with the vertical synch signals of the stereoscopic video signal presented on the monitor 24. Masks or glasses of this type are commercially available, as are many equivalent viewing systems.

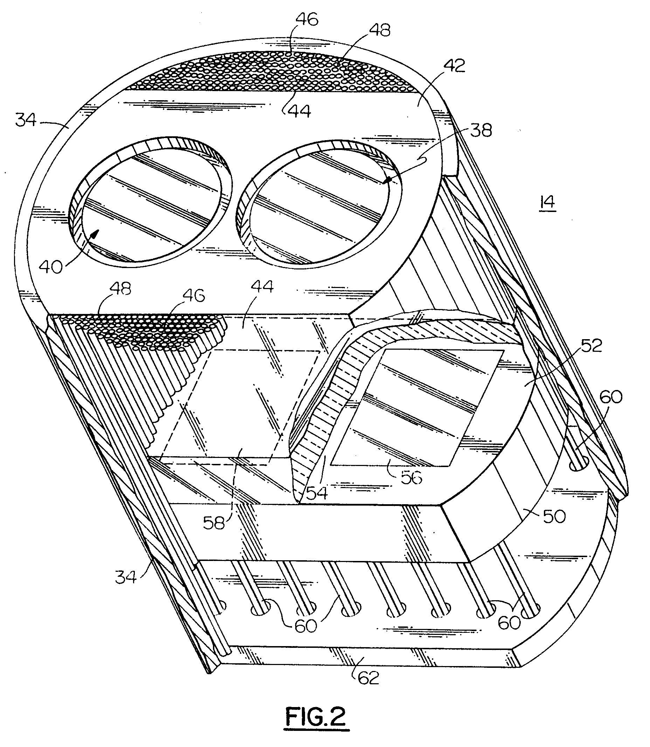

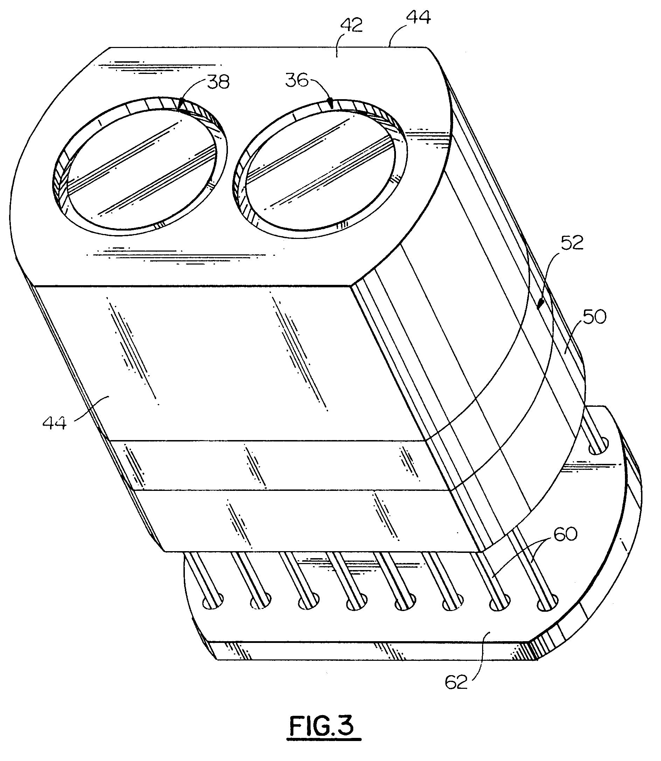

Details of the miniature stereo video camera 14 are shown in Figs. 2 and 3. As shown in Fig. 2, the distal tip of the insertion tube 12 comprises a stainless steel tubular sheath 34, here with an outer diameter of twelve millimeters.

The camera 14 has a stereoscopic dual objective lens assembly 36 containing a left lens group or cell 38 and a right lens group or cell 40. These lens cells 38, 40 are aligned axially, that is they have their optic axes parallel to each other and also parallel to the axis of the insertion tube 12. The lens groups or cells 38, 40 are contained in an aluminum lens housing 42, here with a cross section that is arcuate at its lateral sides, and with flat sides 44 above and below the lens cells i.e., in planes parallel to the plane that contains the optic axes of the lens cells. The lateral sides match the inside curvature of the stainless steel sheath 34 at a cross axis in a plane that contains the optic axes of the right and left lens cells 38, 40. The flat sides 44 together with the sheath 34 define crescent-shaped upper and lower voids or passages 46. It is in these passages 46 that distal ends 48 of the optical fiber bundle are fanned out and reposed. Each crescent shaped bundle end 48 can be covered in suitable light blocking means (such as foil wrap, black paint, etc.) to prevent stray light from escaping to the imaging system.

Proximally of the lens assembly 36 is a solid state imaging device 50, preferably a full-color CCD imager. A flat glass spacer 52 is situated between the lens housing 42 and the imaging device 50.

In this embodiment, the device 50 has a single semiconductor substrate 54 that has both a left active imaging area 56 and a right active imaging area 58. The imaging areas are flat and coplanar, and have identical optical and electrical attributes. The device has conductor pins 60 that project proximally and connect it to a hybrid board 62 within the insertion tube 12. A not-shown conductor cable carries left and right video output signals through the tube 12, handle 16, and umbilical 18 to the modular connector 20 where the video output signals are processed to produce a monitor-ready stereo video signal. As mentioned before, this signal can be similar to a standard (NTSC or PAL) signal, but operated at double the usual field rate. Alternately, a high-density, high-resolution video system can be employed here.

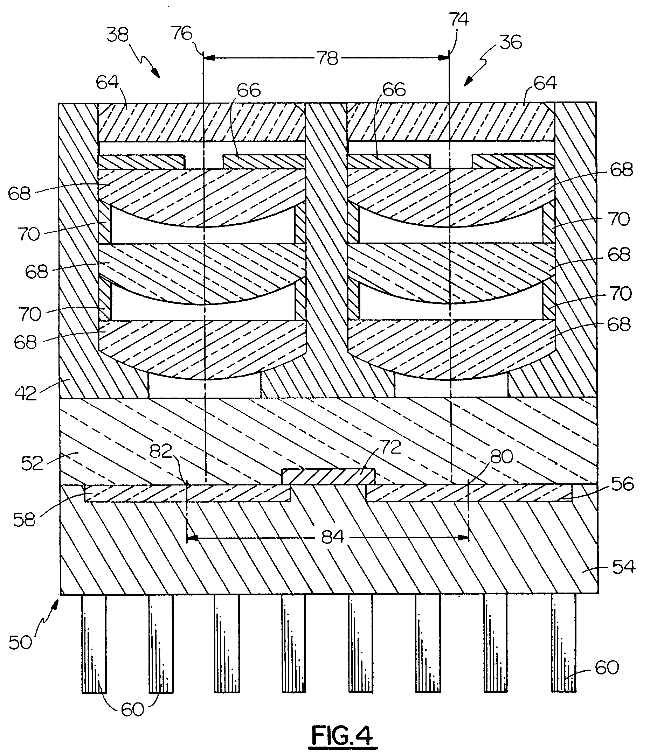

As shown in Fig. 4, the left and right lens cells 38, 40 each have a flat plano-plano lens 64 followed by a diaphragm 66 with a predetermined aperture. This is followed by a series of focussing lenses 68 with alternating spacing rings 70.

On the glass spacer 52 there is an opaque strip or zone 72. This strip extends vertically, i.e., perpendicular to the plane that contains optic axes 74, 76 of the respective lens groups 56, 58. In this view, the strip extends perpendicular to the plane of the page. This strip 72 is intended to block light from the left lens group 38 from imaging on the right imaging area 58 and to block light from the right lens group 40 from imaging on the left imaging area 56. Alternatively, the strip 72 can be a vertical opaque bar.

As also shown here, in this embodiment there is a predetermined distance 78 or pupil distance between the optic axes 74, 76 of the two lens cells 38, 40. The imaging areas 56, 58 have respective centers 80 and 82, with a spacing 84 therebetween that is somewhat greater than the pupil distance 78. In a practical embodiment the pupil distance 78 can be 0.190 inches and the spacing 84 can be 0.210 inches, which produces an image convergence distance of about one and one-half inches in front of the insertion tube tip. The video signals can be electronically manipulated to move the respective images left or right so as to change the convergence distance for viewing of a given target.

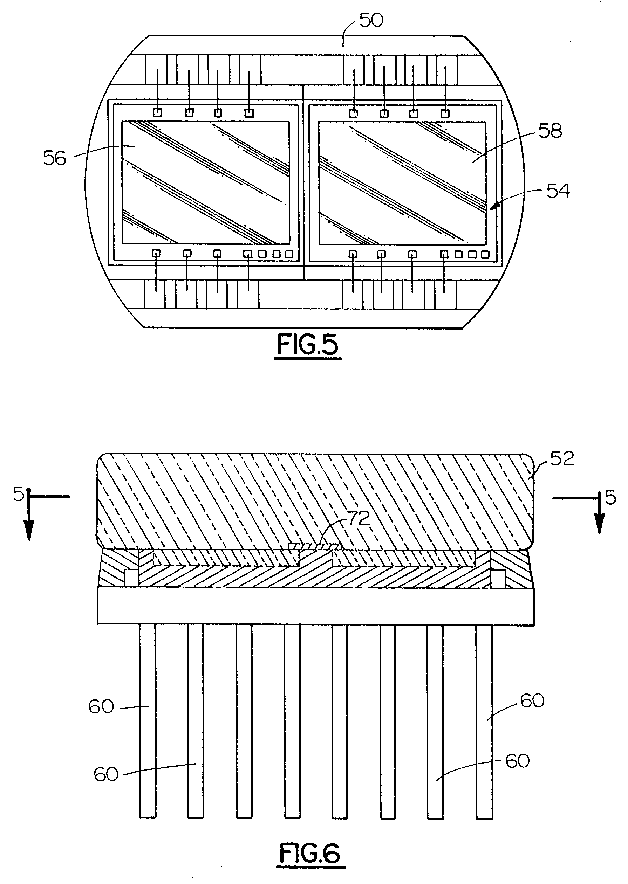

Figs. 5 and 6 show details of a practical embodiment of the imager 50 and spacer 52, and in particular shows the side-by-side orientation of the active imaging areas 56, 58 on the common substrate 54.

The images from the respective imaging areas 56, 58 are read as lines of pixels in the processing circuitry (not shown) contained, e.g. in the modular connector 20. The centers of the left and right images appearing on the monitor 24 can be made to coincide. This can be done automatically based on, e.g., brightness values of features in the image, or can be done under manual control, e.g. with a joystick control. Shifting the image centers changes the convergency distance, as aforementioned, and this effective image shift information can be used for automatically computing distance to the target. This distance information can be displayed on the monitor.

Because the image centers are off the optic axes of the respective lens cells 38, 40, the aperture or opening in the diaphragm need not be strictly circular. The apertures should be selected to achieve a good depth of field, but yet to admit enough illumination for good imaging.

Placing both imaging areas in the same flat plane and orienting the lens cells axially, rather than at an angle, avoid fisheye elongation at edges of the reproduced image. However, in other embodiments, the two image areas can be in planes that are angled, and the two lens cells can be similarly angled.

In this embodiment the insertion tube sheath 34 has a diameter of 12 mm, the lens housing or block 42 is dimensioned 0.452 by 0.304 inches, and the lens cells each have lens diameters of 0.175 inches, and have a preset focal distance of 1.5 inches. The imager device 50 is of the interline transfer CCD type, and the active imaging areas 56, 58 each have a pixel resolution of 510 x 488 (active).

While this invention has been described with reference to one preferred embodiment, it is apparent that the invention is not limited to that precise embodiment. Rather many modifications and variations will present themselves to those skilled in the art without departing from the scope and spirit of this invention, as defined in the appended claims.

## Claims

1. Stereo imaging assembly for an endoscopic or laparascopic probe (10) of the type that has an elongated insertion tube (12) at a distal end of which the imaging assembly is disposed, said imaging assembly characterized by an objective lens assembly (36) situated at the distal end of the insertion tube (12) including left and right lens cells (38,40) disposed side by side in said objective lens assembly and having respective optic axes parallel to one another and parallel to the axis of the insertion tube at its distal end, said optic axes having a predetermined pupil distance therebetween; and an electronic dual imager device 50, disposed in said insertion tube proximate to said objective lens assembly and having left and right active image areas (56,58) formed side by side thereon in a common image plane, said active image areas having respective centers that are separated from one another by a predetermined center-to-center spacing, said center-to-center spacing being greater than said pupil distance.

2. The stereo imaging assembly of claim 1 wherein said insertion tube has a tubular wall (34) of circular cross section, and said imager device and said lens assembly have arcuate side faces that match an inside curvature of said tubular wall at a cross axis containing said left and right axes, and have a first flat face (44) above and parallel to said cross axis, said first flat face and said tubular wall defining a crescent shaped passage; (46) and further comprising an illumination optical conduit extending from a proximal light source through said insertion tube and having a distal end (48) reposed in said crescent shaped passage.

3. The stereo imaging assembly of claim 2 wherein said imager device and said lens assembly have a second flat face below and parallel to said cross axis, which with said tubular wall defines an additional distal end reposed in said additional crescent shaped passage, and said optical conduit has an additional distal end reposed in said additional crescent-shaped passage.

4. The stereo imaging assembly of claim 2 wherin said distal end of the optical conduit is coated with light blocking means to prevent stray light leaking therefrom from passing to said imager device.

5. The stereo imaging assembly according to claim 1 further comprising a transparent space (52) interposed between said objective lens assembly and said imager device.

6. The stereo imaging assembly according to claim 5 wherein said spacer includes an opaque barrier stripe (72) oriented along a zone normal to said optical axes and midway therebetween to block light passing through each of the lens cells from falling on the imaging area associated with the other lens cell.
